# EUROPEAN PATENT SPECIFICATION

(11) **EP 4 340 831 B1**
(45) Date of publication and mention of the grant of the patent: **07.10.2026**
(21) Application number: 22731094.3
(22) Date of filing: 19.05.2022
(51) Int. Cl.: A61K 31/4184, A61P 21/00, A61P 43/00

(54) **KL1333 FOR USE IN MEDICINE**
KL1333 ZUR VERWENDUNG IN DER MEDIZIN
KL1333 DESTINÉ À ÊTRE UTILISÉ EN MÉDECINE

(30) Priority: 19.05.2021 DK PA202170253
(43) Date of publication of application: 27.03.2024
(60) Divisional application: 26201116.6
(73) Proprietor: Abliva AB, 223 81 Lund (SE); Yungjin Pharm. Co., Ltd., Seoul 05510 (KR)
(72) Inventor: HANSSON, Magnus Joakim, 261 33 Landskrona (SE); HUGERTH, Matilda, 237 35 Bjärred (SE); GRÖNBERG, Alvar, 741 42 Knivsta (SE)
(74) Representative: Reddie & Grose LLP
(86) International application number: PCT/EP2022/063583
(87) International publication number: WO 2022/243435

(56) References cited:
- ANONYMOUS: "KL 1333 ready for pivotal Phase II/III trial", 27 May 2021 (2021-05-27), Edison Group, pages 1 - 13, XP55948019, Retrieved from the Internet <URL:https://www.edisongroup.com/wp-content/uploads/2021/05/Abliva-KL1333-ready-for-pivotal-Phase-IIIII-trial.pdf> [retrieved on 20220802]
- ANONYMOUS: "Reduce Fatigue by Addressing Mitochondrial Dysfunction | IFM", 21 September 2020 (2020-09-21), XP55947990, Retrieved from the Internet <URL:https://www.ifm.org/news-insights/reduce-fatigue-by-addressing-mitochondrial-dysfunction/> [retrieved on 20220802]
- SEO KANG-SIK ET AL: "KL1333, a Novel NAD+ Modulator, Improves Energy Metabolism and Mitochondrial Dysfunction in MELAS Fibroblasts", FRONTIERS IN NEUROLOGY, vol. 9, 1 January 2018 (2018-01-01), pages 1 - 12, XP55948026, Retrieved from the Internet <URL:https://www.ncbi.nlm.nih.gov/pmc/articles/PMC6041391/pdf/fneur-09-00552.pdf> DOI: 10.3389/fneur.2018.00552
- COEN PAUL M. ET AL: "Mitochondria as a Target for Mitigating Sarcopenia", FRONTIERS IN PHYSIOLOGY, vol. 9, 1 January 2019 (2019-01-01), pages 1 - 15, XP55948000, Retrieved from the Internet <URL:https://www.ncbi.nlm.nih.gov/pmc/articles/PMC6335344/pdf/fphys-09-01883.pdf> DOI: 10.3389/fphys.2018.01883
- PARIKH SUMIT ET AL: "Diagnosis and management of mitochondrial disease: a consensus statement from the Mitochondrial Medicine Society", vol. 17, no. 9, 1 September 2015 (2015-09-01), pages 689 - 701, XP009505121, ISSN: 1098-3600, Retrieved from the Internet <URL:http://www.nature.com/articles/gim2014177> [retrieved on 20141211], DOI: 10.1038/GIM.2014.177
- SEO KANG-SIK ET AL: "KL1333, a Novel NAD+ Modulator, Improves Energy Metabolism and Mitochondrial Dysfunction in MELAS Fibroblasts", FRONTIERS IN NEUROLOGY, vol. 9, 1 January 2018 (2018-01-01), pages 1 - 12, XP055948026, Retrieved from the Internet <URL:https://www.ncbi.nlm.nih.gov/pmc/articles/PMC6041391/pdf/fneur-09-00552.pdf> DOI: 10.3389/fneur.2018.00552
- ANONYMOUS: "Reduce Fatigue by Addressing Mitochondrial Dysfunction | IFM", 21 September 2020 (2020-09-21), XP055947990, Retrieved from the Internet <URL:https://www.ifm.org/news-insights/reduce-fatigue-by-addressing-mitochondrial-dysfunction/> [retrieved on 20220802]
- COEN PAUL M. ET AL: "Mitochondria as a Target for Mitigating Sarcopenia", FRONTIERS IN PHYSIOLOGY, vol. 9, 1 January 2019 (2019-01-01), pages 1 - 15, XP055948000, Retrieved from the Internet <URL:https://www.ncbi.nlm.nih.gov/pmc/articles/PMC6335344/pdf/fphys-09-01883.pdf> DOI: 10.3389/fphys.2018.01883
- ANONYMOUS: "KL 1333 ready for pivotal Phase II/III trial", 27 May 2021 (2021-05-27), Edison Group, pages 1 - 13, XP055948019, Retrieved from the Internet <URL:https://www.edisongroup.com/wp-content/uploads/2021/05/Abliva-KL1333-ready-for-pivotal-Phase-IIIII-trial.pdf> [retrieved on 20220802]

## Description

### FIELD OF THE INVENTION

The present invention is set out in the appended set of claims and relates to the medical use of KL1333 in the treatment of fatigue or muscle weakness, wherein said fatigue or muscle weakness is a manifestation associated with a mitochondrial disease.

### BACKGROUND OF THE INVENTION

Fatigue and muscle weakness are often associated with or caused by specific diseases. Mitochondria are important organelles that generate most of the energy required by the human body in the form of adenosine triphosphate (ATP) via the electron transport chain. Primary mitochondrial diseases are generally triggered by dysfunction of the electron transport chain, resulting in disorders in mitochondrial energy production or excessive reactive oxygen species (ROS) generation. Hundreds of primary mitochondrial diseases are known, including Mitochondrial Encephalomyopathy with Lactic Acidosis and Stroke-like episodes (MELAS), Leber Hereditary Optic Neuropathy, Myoclonic Epilepsy with Ragged-Red Fibers, and Leigh syndrome. The disorders can manifest differently depending on the organs affected and have historically been viewed as clinical syndromes, and more recently as disease spectra caused by genetic defects affecting mitochondrial function. An estimated 125 in every 1,000,000 people suffer from primary mitochondrial disease. Clinical manifestations of primary mitochondrial diseases cover a wide spectrum of phenotypes including serious and life-threatening conditions such as organ failure, cardiorespiratory arrest, intracranial haemorrhage, leukaemia/lymphoma, myocardial ischaemia, intestinal obstruction, and immune deficiency, as well as an even wider range of other potentially debilitating conditions.

The Institute for Functional Medicine, "Reduce Fatigue by Addressing Mitochonrial Dysfunction", 21 September 2020, describes how functional medicine can help patients with fatigue. Coen Paul M. et al., "Mitochondria as a Target for Mitigating Sarcopena", January 2019, describes how Sarcopenia may be treated or mitigated.

At present there are no approved medicine for primary mitochondrial diseases. Thus, there is a need for identifying drug substances that are effective against mitochondrial diseases and/or effective against the disorders or diseases associated with mitochondrial diseases.

### DETAILED DESCRIPTION

KL1333 is a novel compound under development for primary mitochondrial diseases. KL1333 acts as a substrate for NAD(P)H:dehydrogenase [quinone]1 (NQO1), which produces nicotinamide adenine dinucleotide (oxidized form; NAD⁺) by transferring 2 electrons to KL1333 using nicotinamide adenine dinucleotide (reduced form; NADH) as a cofactor. KL1333 transfers these electrons to the mitochondrial electron transport system, directly promoting ATP production. Additionally, the elevated NAD⁺ levels lead to activation of mitochondrial biogenesis pathways, such as sirtuin 1 (SIRT1), 5'-adenosine monophosphate-activated protein kinase (AMPK), and peroxisome proliferator-activated receptor gamma coactivator 1-alpha (PGC-1α), thereby improving mitochondrial function.

KL1333 has in preclinical models been demonstrated to increase mitochondrial energy output and to have long-term beneficial effect on energy metabolism. In the clinical study reported herein, KL1333 has demonstrated to decrease fatigue and to strengthen muscle function.

The present invention is set out in the appended set of claims and relates to
i) KL1333 for use in the treatment of fatigue such as fatigue syndrome and fatigue associated with a mitochondrial disease,
ii) KL1333 for use in strengthening of muscle function such as muscle weakness associated with a mitochondrial disease,
iii) KL1333 for use in a dosage regime for the treatment of one or more of fatigue, muscle weakness, wherein said fatigue or muscle weakness is a manifestation associated with a mitochondrial disease, the dosage regime comprising
   i) administering to a subject suffering from fatigue, muscle weakness or a mitochondrial disease in a range of from 25 to 100 mg KL1333 daily for a time period of from 2 to 10 days to obtain steady state concentrations of KL1333 in the blood,
   ii) measuring the blood or plasma concentration of KL1333 expressed as AUC (area under the curve), C min, and if AUC is below 3,000 h*ng/mL or Cmin is 65 ng/mL or less, and measuring of plasma concentration is preferred,
   iii) adjusting the daily dose to obtain steady state concentrations of KL1333 in the blood or plasma corresponding to an AUC (area under the curve) of at least 3,000 h*ng/mL and/or Cmin of at least 65 ng/ml, or such that AUC is at least 3,500 h*ng/mL and/or Cmin is at least 77 ng/mL, or such that AUC is at least 4,000 h*ng/mL and/or Cmin is at least 88 ng/mL, or such that AUC is at least 4,500 h*ng/mL and/or Cmin is at least 100 ng/mL, or such that AUC is in a range of from 4,000 to 12,000 h*ng/mL and/or Cmin is in a range of from 88 ng/mL to 275 ng/mL at day 10 after adjustment of the dose, and measuring of plasma concentration is preferred.

The following aspects (iv) and (v) are not part of the invention:
iv) use of blood lactate (mM)/pyruvate (mM) ratio as biomarker of KL1333 treatment effect, wherein a decrease in ratio indicates that the treatment is effective, and
v) use of serum niacinamide and/or xanthine as early response biomarkers of KL1333 treatment effect, wherein an increase in the ratio of niacinamide and/or xanthine concentrations indicates that the treatment is effective and the ratio of niacinamide and/or xanthine concentrations is (serum concentration at test day)/(serum concentration at the start of the treatment).

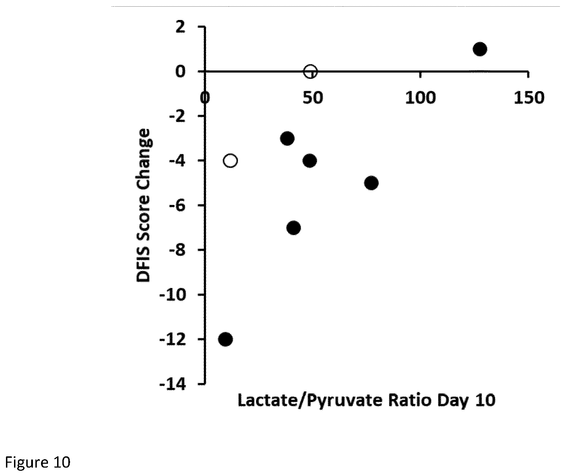

In conclusion, blood lactate/pyruvate ratio, niacinamide and xanthine may be used as biomarkers of KL1333 treatment effect in PMD patients. To that end, it was found that patients with the strongest decrease in the DFIS fatigue score Day 10 of KL1333 treatment also had a low lactate/pyruvate ratio in serum (Figure 10).

In the present context, the term Cmin is the minimum blood or plasma concentration at steady state and Ctrough is the blood or plasma concentration just before next dose is given. Measuring of plasma concentration is preferred.

Regarding the decrease in blood lactate (mM)/pyruvate (mM) that is indicative for an effective treatment, a decrease of at least 10% such as at least 15%, at least 20%, at least 25%, at least 30%, at least 35%, at least 40%, at least 45% or at least 50% based on a start value is indicative of an effective treatment. The start value may be the value before treatment is initiated or it may be a a time point after the treatment has started so that the efficiency of the treatment is followed over time. Regarding the increase in serum niacinamide, the increase may be 2 times or more such as 3 times or more from the start value as defined above. Regarding the increase in serum xanthine, the increase may be 20% or more such as 25% or more or 30% or more.

KL-1333 is a drug substance with a molecular mass of 240.26 g/mol. It is a light red to reddish brown, non-hygroscopic, crystalline powder that is practically insoluble in water. It is manufactured via multi-step chemical synthesis. The molecular structure is shown below:

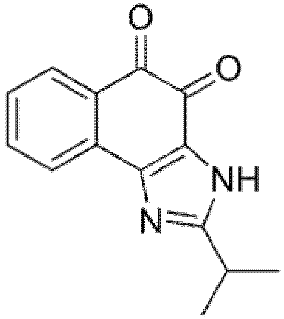

KL1333 was shown to be a more potent substrate to NQO1 than other NQO1 active compounds developed for primary mitochondrial disease (ie, idebenone). In cellular models, including cells derived from patients with MELAS, KL1333 demonstrated increased ATP; decreased ROS; decreased lactic acid; increased NAD⁺; activation of SIRT1, AMPK, and PGC-1α; and improved mitochondrial oxidative phosphorylation function.

In the clinical study reported herein (KL1333 2018-102), KL1333 or placebo were administered to 64 healthy volunteers and 8 patients with genetically confirmed mitochondrial disease. There were no serious adverse events in the study. There were no apparent treatment- or dose-related trends in the mean or individual subject clinical chemistry, hematology, or urinalysis data during the study. There were no apparent treatment- or dose-related trends in vital signs measurements, 12-lead ECGs, or physical examinations in this study. KL1333 was well tolerated when administered as single oral doses of 25 mg with or without food in healthy subjects. In healthy subjects, QD doses of 25 to 75 mg were well tolerated, doses of 150 mg were tolerated, and doses of 250 mg were poorly tolerated due to GI TEAEs. Daily doses of 150 mg KL1333 were better tolerated as BID or TID doses versus QD doses, with a reduced frequency and intensity of GI-related adverse events. KL1333 was well tolerated when administered as multiple oral QD doses of 50 mg for 10 days to patients with primary mitochondrial disease. In the clinical outcome assessments of primary mitochondrial disease patients in the study, KL1333 has proven to be effective in combatting fatigue and muscle weakness.

### Fatigue

As demonstrated herein KL1333 is effective against fatigue. Fatigue may be in the form of chronic fatigue syndrome or it may be associated with other diseases such as mitochondrial disease e.g. primary mitochondrial diseases.

Fatigue is a feeling of tiredness. It may be a sudden or gradual in onset. It is a normal phenomenon if it follows prolonged physical or mental activity, and resolves completely with rest. However, it may be a symptom of a medical condition if it is prolonged, severe, progressive, or occurs without provocation.

Physical fatigue is the transient inability of muscles to maintain optimal physical performance, and is made more severe by intense physical exercise. Physical fatigue, or muscle fatigue, can be caused by a lack of energy in the mudcle, by a decrease of the efficiency of the neuromuscular junction or by a reduction of the drive originating from the central nervous systen. The central component of fatigue is triggered by an increase of the level of serotonin in the central nervous system. Physical fatigue may be caused by a neuromuscular disease.

Mental fatigue is a transient decrease in maximal cognitive performance resulting from prolonged periods of cognitive activity. It can manifest as somnolence, lethargy or directed attention fatigue.

Neurological fatigue may occour in patient with multiple sclerosis. Such patients often experience a form of overwhelming lassitude or tiredness.

Chronic fatigue is fatigue lasting for at least six consecutive months. Chronic fatigue is a symptom of many diseases and conditions. Some major diseases that are associated with fatigue include:
i) Autoimmune disease such as celiac disease, lupus, multiple sclerosis, Sjögren's syndrome and spondyloarthropathy,
ii) blood disorders such as anemia and hemochromatosis,
iii) cancer (denoted cancer fatigue)
iv) chronic fatigue syndrome (CFS)
v) substance use disorders including alcohol use disorder
vi) depression and other metal disorders
vii) developmental disorders such as autism spectrum disorder
viii) eating disorders
ix) endocrine disease or metabolic disorders, diabetes mellitus, hypothyrodism and Addison's disease,
x) fibromyalgia,
xi) Gulf War syndrome,
xii) heart failure
xiii) HIV
xiv) Idiopathic chronic fatigue (ICF)
xv) inborn errors of metabolism such as fructose malabsorption
xvi) infectious diseases such as infectious mononucleosis or tuberculosis
xvii) irritable bowel syndrome
xviii) kidney diseases e.g. acute renal failure, chronic renal failure,
xix) leukemia or lymphoma
xx) liver failure or liver diseases such as hepatitis
xxi) Lyme diseaase
xxii) neurological disorders such as narcolepsy, Parkinson's disease, Postural Orthostatic Tachycardia Syndrome and post-concussion syndrome
xxiii) physical trauma and other pain-causing conditions such as arthirtis
xxiv) sleep deprivation or sleep disorders
xxv) spring fever
xxvi) stroke
xxvii) thyroid disease
xxviii) uremia
xxix) mitochondrial diseases.

### Muscle weakness

Muscle weakness is a lack of muscle strength. True muscle weakness is a primary symptom of a variety of skeletal muscle disease. Muscle weakness may be neuromuscular fatigue that can be classified as either central or periferal depending on its cause. Central muscle fatigue manifests as an overall sense of energy deprivation, while peripheral muscle fatigue manifests as a local, muscle-specific inability to work.

Muscle weakness is commonly due to lack of exercise, ageing, or muscle injury. It can also occur with long-term conditions such as diabetes and heart disease, stroke, depression, fibromyalgia, chronic fatigue syndrome, polymyositis, inflammatory myopathy, mitochondrial diseases, neuromuscular disorders such as muscular dystrophies, multiple sclerosis, Graves' disease, myasthenia gravis and Guillain-Barrë syndrome.

### Mitochondrial diseases

KL1333 is used in the treatment of fatigue or muscle weakness, wherein said fatigue or muscle weakness is a manifestation associated with a mitochondrial disease. Mitochondrial diseases are selected from the following:
- Alpers Disease (Progressive Infantile Poliodystrophy)
- Amyotrophic lateral sclerosis (ALS)
- Autism
- Barth syndrome (Lethal Infantile Cardiomyopathy)
- Beta-oxidation Defects
- Bioenergetic metabolism deficency
- Carnitine-Acyl-Carnitine Deficiency
- Carnitine Deficiency
- Creatine Deficiency Syndromes (Cerebral Creatine Deficiency Syndromes (CCDS) includes: Guanidinoaceteate Methyltransferase Deficiency (GAMT Deficiency), L-Arginine:Glycine Amidinotransferase Deficiency (AGAT Deficiency), and SLC6A8-Related Creatine Transporter Deficiency (SLC6A8 Deficiency).
- Co-Enzyme Q10 Deficiency
- Complex I Deficiency (NADH dehydrogenase (NADH-CoQ reductase) deficiency)
- Complex II Deficiency (Succinate dehydrogenase deficiency)
- Complex III Deficiency (Ubiquinone-cytochrome c oxidoreductase deficiency)
- Complex IV Deficiency/COX Deficiency (Cytochrome c oxidase deficiency is caused by a defect in Complex IV of the respiratory chain)
- Complex V Deficiency (ATP synthase deficiency)
- COX Deficiency
- CPEO (Chronic Progressive External Ophthalmoplegia Syndrome)
- CPT I Deficiency
- CPT II Deficiency
- Friedreich's ataxia (FRDA or FA)
- Glutaric Aciduria Type II
- KSS (Kearns-Sayre Syndrome)
- Lactic Acidosis
- LCAD (Long-Chain Acyl-CoA Dehydrogenase Deficiency)
- LCHAD
- Leigh Disease or Syndrome (Subacute Necrotizing Encephalomyelopathy)
- LHON (Leber's hereditary optic neuropathy)
- Luft Disease
- MCAD (Medium-Chain Acyl-CoA Dehydrogenase Deficiency)
- MELAS (Mitochondrial Encephalomyopathy Lactic Acidosis and Strokelike Episodes)
- MERRF (Myoclonic Epilepsy and Ragged-Red Fiber Disease)
- MIDD (Maternally inherited Diabetes and Deafness)
- MIRAS (Mitochondrial Recessive Ataxia Syndrome)
- Mitochondrial Cytopathy
- Mitochondrial DNA Depletion
- Mitochondrial Encephalopathy includes: Encephalomyopathy, Encephalomyelopathy
- Mitochondrial Myopathy
- MNGIE (Myoneurogastointestinal Disorder and Encephalopathy)
- NARP (Neuropathy, Ataxia, and Retinitis Pigmentosa)
- Neurodegenerative disorders associated with Parkinson's, Alzheimer's or Huntington's disease
- Pearson Syndrome
- Pyruvate Carboxylase Deficiency
- Pyruvate Dehydrogenase Deficiency
- POLG Mutations
- Respiratory Chain Deficiencies
- SCAD (Short-Chain Acyl-CoA Dehydrogenase Deficiency)
- SCHAD ( Short Chain L-3-Hydroxyacyl-CoA Dehydrogenase (SCHAD) Deficiency, also referred to as 3-Hydroxy Acyl CoA Dehydrogenase Deficiency HADH
- VLCAD (Very Long-Chain Acyl-CoA Dehydrogenase Deficiency)
- Diabetes
- Acute starvation
- Endotoxemia
- Sepsis
- Systemic inflammation response syndrome (SIRS)
- Multiple organ failure

With reference to information from the web-page of United Mitochondrial Disease Foundation (www.umdf.org), some of the above-mentioned diseases are discussed in more details in the following:
*Complex I deficiency:* Inside the mitochondrion is a group of proteins that carry electrons along four chain reactions (Complexes I-IV), resulting in energy production. This chain is known as the Electron Transport Chain. A fifth group (Complex V) churns out the ATP. Together, the electron transport chain and the ATP synthase form the respiratory chain and the whole process is known as oxidative phosphorylation or OXPHOS.

Complex I, the first step in this chain, is the most common site for mitochondrial abnormalities, representing as much as one third of the respiratory chain deficiencies. Often presenting at birth or in early childhood, Complex I deficiency is usually a progressive neurodegenerative disorder and is responsible for a variety of clinical symptoms, particularly in organs and tissues that require high energy levels, such as brain, heart, liver, and skeletal muscles. A number of specific mitochondrial disorders have been associated with Complex I deficiency including: Leber's hereditary optic neuropathy (LHON), MELAS, MERRF, and Leigh Syndrome (LS). MELAS stands for (mitochondrial encephalomyopathy, lactic acidosis, and stroke-like episodes) and MERRF stand for myoclonic epilepsy with ragged red fibers.

LHON is characterized by blindness which occurs on average between 27 and 34 years of age; blindness can develop in both eyes simultaneously, or sequentially (one eye will develop blindness, followed by the other eye two months later on average). Other symptoms may also occur, such as cardiac abnormalities and neurological complications.

There are three major forms of Complex I deficiency:
i) Fatal infantile multisystem disorder - characterized by poor muscle tone, developmental delay, heart disease, lactic acidosis, and respiratory failure.
ii) Myopathy (muscle disease) - starting in childhood or adulthood, and characterized by weakness or exercise intolerance.
iii) Mitochondrial encephalomyopathy (brain and muscle disease) - beginning in childhood or adulthood and involving variable symptom combinations which may include: eye muscle paralysis, pigmentary retinopathy (retinal color changes with loss of vision), hearing loss, sensory neuropathy (nerve damage involving the sense organs), seizures, dementia, ataxia (abnormal muscle coordination), and involuntary movements. This form of Complex I deficiency may cause Leigh Syndrome and MELAS.

Most cases of Complex I deficiency result from autosomal recessive inheritance (combination of defective nuclear genes from both the mother and the father). Less frequently, the disorder is maternally inherited or sporadic and the genetic defect is in the mitochondrial DNA.

Treatment: As with all mitochondrial diseases, there is presently no cure for Complex I deficiency. A variety of treatments, which may or may not be effective, can include such metabolic therapies as: riboflavin, thiamine, biotin, co-enzyme Q10, carnitine, and ketogenic diet. Therapies for the infantile multisystem form have been unsuccessful.

The clinical course and prognosis for Complex I patients is highly variable and may depend on the specific genetic defect, age of onset, organs involved, and other factors.

*Complex III Deficiency:* The symptoms include four major forms:
i) Fatal infantile encephalomyopathy, congenital lactic acidosis, hypotonia, dystrophic posturing, seizures, and coma. Ragged-red fibers in muscle tissue are common.
ii) Encephalomyopathies of later onset (childhood to adult life): various combinations of weakness, short stature, ataxia, dementia, hearing loss, sensory neuropathy, pigmentary retinopathy, and pyramidal signs. Ragged-red fibers are common. Possible lactic acidosis.
iii) Myopathy, with exercise intolerance evolving into fixed weakness. Ragged-red fibers are common. Possible lactic acidosis.
iv) Infantile histiocytoid cardiomyopathy.

*Complex IV Deficiency* / *COX Deficiency:* The symptoms include two major forms:
1. Encephalomyopathy: Typically normal for the first 6 to 12 months of life and then show developmental regression, ataxia, lactic acidosis, optic atrophy, ophthalmoplegia, nystagmus, dystonia, pyramidal signs, and respiratory problems. Frequent seizures. May cause Leigh Syndrome
2. Myopathy: Two main variants:
   1. Fatal infantile myopathy: may begin soon after birth and accompanied by hypotonia, weakness, lactic acidosis, ragged-red fibers, respiratory failure, and kidney problems.
   2. Benign infantile myopathy: may begin soon after birth and accompanied by hypotonia, weakness, lactic acidosis, ragged-red fibers, respiratory problems, but (if the child survives) followed by spontaneous improvement.

KSS (Kearns-Sayre Syndrome): KSS is a slowly progressive multi-system mitochondrial disease that often begins with drooping of the eyelids (ptosis). Other eye muscles eventually become involved, resulting in paralysis of eye movement. Degeneration of the retina usually causes difficulty seeing in dimly lit environments.

KSS is characterized by three main features:
- typical onset before age 20 although may occur in infancy or adulthood
- paralysis of specific eye muscles (called chronic progressive external ophthalmoplegia - CPEO)
- degeneration of the retina causing abnormal accumulation of pigmented (colored) material (pigmentary retinopathy).

In addition, one or more of the following conditions is present:
- block of electrical signals in the heart (cardiac conduction defects)
- elevated cerebrospinal fluid protein
- incoordination of movements (ataxia).

Patients with KSS may also have such problems as deafness, dementia, kidney dysfunction, and muscle weakness. Endocrine abnormalities including growth retardation, short stature, or diabetes may also be evident.

KSS is a rare disorder. It is usually caused by a single large deletion (loss) of genetic material within the DNA of the mitochondria (mtDNA), rather than in the DNA of the cell nucleus. These deletions, of which there are over 150 species, typically arise spontaneously. Less frequently, the mutation is transmitted by the mother.

As with all mitochondrial diseases, there is no cure for KSS.

Treatments are based on the types of symptoms and organs involved, and may include: Coenzyme Q10, insulin for diabetes, cardiac drugs, and a cardiac pacemaker which may be life-saving. Surgical intervention for drooping eyelids may be considered but should be undertaken by specialists in ophthalmic surgical centers.

KSS is slowly progressive and the prognosis varies depending on severity. Death is common in the third or fourth decade and may be due to organ system failures.

*Leigh Disease or Syndrome* (Subacute Necrotizing Encephalomyelopathy): Symptoms: Seizures, hypotonia, fatigue, nystagmus, poor reflexes, eating and swallowing difficulties, breathing problems, poor motor function, ataxia.

Causes: Pyruvate Dehydrogenase Deficiency, Complex I Deficiency, Complex II Deficiency, Complex IV/COX Deficiency, NARP.

Leigh's Disease is a progressive neurometabolic disorder with a general onset in infancy or childhood, often after a viral infection, but can also occur in teens and adults. It is characterized on MRI by visible necrotizing (dead or dying tissue) lesions on the brain, particularly in the midbrain and brainstem.

The child often appears normal at birth but typically begins displaying symptoms within a few months to two years of age, although the timing may be much earlier or later. Initial symptoms can include the loss of basic skills such as sucking, head control, walking and talking. These may be accompanied by other problems such as irritability, loss of appetite, vomiting and seizures. There may be periods of sharp decline or temporary restoration of some functions. Eventually, the child may also have heart, kidney, vision, and breathing complications.

There is more than one defect that causes Leigh's Disease. These include a pyruvate dehydrogenase (PDHC) deficiency, and respiratory chain enzyme defects - Complexes I, II, IV, and V. Depending on the defect, the mode of inheritance may be X-linked dominant (defect on the X chromosome and disease usually occurs in males only), autosomal recessive (inherited from genes from both mother and father), and maternal (from mother only). There may also be spontaneous cases which are not inherited at all.

There is no cure for Leigh's Disease. Treatments generally involve variations of vitamin and supplement therapies, often in a "cocktail" combination, and are only partially effective. Various resource sites include the possible usage of: thiamine, coenzyme Q10, riboflavin, biotin, creatine, succinate, and idebenone. Experimental drugs, such as dichloroacetate (DCA) are also being tried in some clinics. In some cases, a special diet may be ordered and must be monitored by a dietitian knowledgeable in metabolic disorders.

The prognosis for Leigh's Disease is poor. Depending on the defect, individuals typically live anywhere from a few years to the mid-teens. Those diagnosed with Leigh-like syndrome or who did not display symptoms until adulthood tend to live longer.

*MELAS* (Mitochondrial Encephalomyopathy Lactic Acidosis and Stroke-like Episodes): Symptoms: Short statue, seizures, stroke-like episodes with focused neurological deficits, recurrent headaches, cognitive regression, disease progression, ragged-red fibers.

### Cause: Mitochondrial DNA point mutations: A3243G (most common)

MELAS - Mitochondrial Myopathy (muscle weakness), Encephalopathy (brain and central nervous system disease), Lactic Acidosis (build-up of a product from anaerobic respiration), and Stroke-like episodes (partial paralysis, partial vision loss, or other neurological abnormalities).

MELAS is a progressive neurodegenerative disorder with typical onset between the ages of 2 and 15, although it may occur in infancy or as late as adulthood. Initial symptoms may include stroke-like episodes, seizures, migraine headaches, and recurrent vomiting.

Usually, the patient appears normal during infancy, although short stature is common. Less common are early infancy symptoms that may include developmental delay, learning disabilities or attention-deficit disorder. Exercise intolerance, limb weakness, hearing loss, and diabetes may also precede the occurrence of the stroke-like episodes.

Stroke-like episodes, often accompanied by seizures, are the hallmark symptom of MELAS and cause partial paralysis, loss of vision, and focal neurological defects. The gradual cumulative effects of these episodes often result in variable combinations of loss of motor skills (speech, movement, and eating), impaired sensation (vision loss and loss of body sensations), and mental impairment (dementia). MELAS patients may also suffer additional symptoms including: muscle weakness, peripheral nerve dysfunction, diabetes, hearing loss, cardiac and kidney problems, and digestive abnormalities. Lactic acid usually accumulates at high levels in the blood, cerebrospinal fluid, or both.

MELAS is maternally inherited due to a defect in the DNA within mitochondria. There are at least 17 different mutations that can cause MELAS. By far the most prevalent is the A3243G mutation, which is responsible for about 80% of the cases.

There is no cure or specific treatment for MELAS. Although clinical trials have not proven their efficacy, general treatments may include such metabolic therapies as: CoQ10, creatine, phylloquinone, and other vitamins and supplements. Drugs such as seizure medications and insulin may be required for additional symptom management. Some patients with muscle dysfunction may benefit from moderate supervised exercise. In select cases, other therapies that may be prescribed include dichloroacetate (DCA) and menadione, though these are not routinely used due to their potential for having harmful side effects.

The prognosis for MELAS is poor. Typically, the age of death is between 10 to 35 years, although some patients may live longer. Death may come as a result of general body wasting due to progressive dementia and muscle weakness, or complications from other affected organs such as heart or kidneys.

*MERRF* is a progressive multi-system syndrome usually beginning in childhood, but onset may occur in adulthood. The rate of progression varies widely. Onset and extent of symptoms can differ among affected siblings.

The classic features of MERRF include:
- Myoclonus (brief, sudden, twitching muscle spasms) - the most characteristic symptom
- Epileptic seizures
- Ataxia (impaired coordination)
- Ragged-red fibers (a characteristic microscopic abnormality observed in muscle biopsy of patients with MERRF and other mitochondrial disorders) Additional symptoms may include: hearing loss, lactic acidosis (elevated lactic acid level in the blood), short stature, exercise intolerance, dementia, cardiac defects, eye abnormalities, and speech impairment.

Although a few cases of MERRF are sporadic, most cases are maternally inherited due to a mutation within the mitochondria. The most common MERRF mutation is A8344G, which accounted for over 80% of the cases. Four other mitochondrial DNA mutations have been reported to cause MERRF. While a mother will transmit her MERRF mutation to all of her offspring, some may never display symptoms.

As with all mitochondrial disorders, there is no cure for MERRF. Therapies may include coenzyme Q10, L-carnitine, and various vitamins, often in a "cocktail" combination. Management of seizures usually requires anticonvulsant drugs. Medications for control of other symptoms may also be necessary.

The prognosis for MERRF varies widely depending on age of onset, type and severity of symptoms, organs involved, and other factors.

*Maternally inherited diabetes and deafness (MIDD)* is a mitochondrial disorder characterized by maternally transmitted diabetes and sensorineural deafness. The first manifestations may occur at any age, but the disease is usually diagnosed in early adulthood. In most cases, the onset of deafness precedes that of diabetes. The severity of the hearing loss is variable but it is sensorineural, bilateral and progressive, and is more profound at higher frequencies. In most cases, patients present pseudo-type 2 diabetes, with a normal or low body mass index. Pseudo-type 1 diabetes, sometimes with ketoacidosis, is observed in 20% of cases. Diabetic retinopathy is less common in MIDD patients than in those with classic forms of diabetes. In more than 80% of cases, patients develop specific macular pattern dystrophy lesions that are only seen in MIDD patients and are asymptomatic in most cases. Organs with high metabolic activity (muscles, myocardium, kidney, and brain) are frequently affected potentially leading to muscle pain, gastrointestinal tract symptoms, nephropathy, cardiomyopathy, and neuropsychiatric symptoms. In most cases, MIDD is caused by a point mutation in the mitochondrial gene MT-TL1, encoding the mitochondrial tRNA for leucine, and in rare cases in MT-TE and MT-TK genes, encoding the mitochondrial tRNAs for glutamic acid, and lysine, respectively.

*Mitochondrial DNA Depletion:* The symptoms include three major forms:
1. Congenital myopathy: Neonatal weakness, hypotonia requiring assisted ventilation, possible renal dysfunction. Severe lactic acidosis. Prominent ragged-red fibers. Death due to respiratory failure usually occurs prior to one year of age.
2. Infantile myopathy: Following normal early development until one year old, weakness appears and worsens rapidly, causing respiratory failure and death typically within a few years.
3. Hepatopathy: Enlarged liver and intractable liver failure, myopathy. Severe lactic acidosis. Death is typical within the first year.

### Friedreich's ataxia

Friedreich's ataxia (FRDA or FA) an autosomal recessive neurodegenerative and cardiodegenerative disorder caused by decreased levels of the protein frataxin. Frataxin is important for the assembly of iron-sulfur clusters in mitochondrial respiratory-chain complexes. Estimates of the prevalence of FRDA in the United States range from 1 in every 22,000-29,000 people (see www.nlm.nih.gov/medlineplus/ency/article/001411.htm) to 1 in 50,000 people. The disease causes the progressive loss of voluntary motor coordination (ataxia) and cardiac complications. Symptoms typically begin in childhood, and the disease progressively worsens as the patient grows older; patients eventually become wheelchair-bound due to motor disabilities.

In addition to congenital disorders involving inherited defective mitochondria, acquired mitochondrial dysfunction has been suggested to contribute to diseases, particularly neurodegenerative disorders associated with aging like Parkinson's, Alzheimer's, and Huntington's Diseases. The incidence of somatic mutations in mitochondrial DNA rises exponentially with age; diminished respiratory chain activity is found universally in aging people. Mitochondrial dysfunction is also implicated in excitotoxicity, neuronal injury, cerebral vascular accidents such as that associated with seizures, stroke and ischemia.

### General

It should be understood that any feature and/or aspect discussed above in connections with the compounds according to the invention apply by analogy to the methods described herein.

The following figures and examples are provided below to illustrate the present invention. They are intended to be illustrative and are not to be construed as limiting in any way.

### BRIEF DESCRIPTION OF THE FIGURES

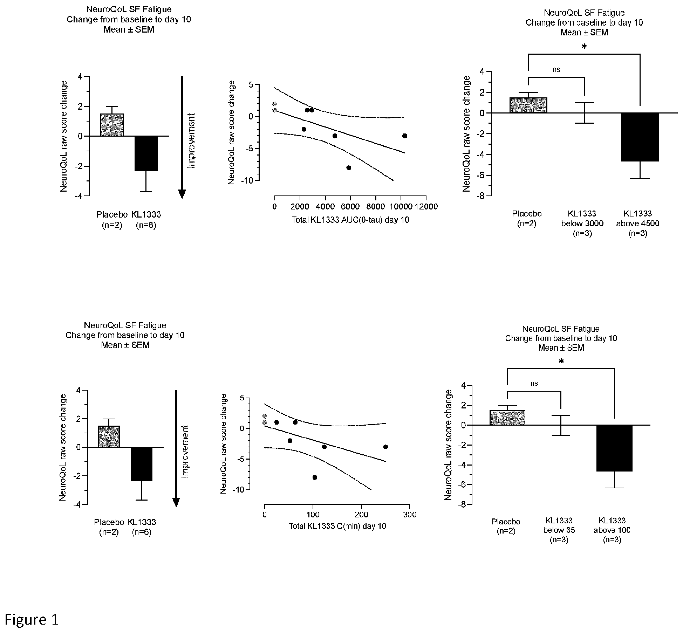

Figure 1 show data results from NeuroQoL Short Form Fatigue. Debilitating fatigue is considered the most important symptom to improve from mitochondrial disease patients.
   Left graph demonstrate mean changes in KL1333 and placebo, with improvement in KL1333 group, but not placebo-treated. Middle graph show correlation between effect sizes (change from baseline to day 10) and exposure levels, Total KL1333 AUC(0-tau) or C(min) at day 10 (h*ng/mL). The right graph is the same data, but actively treated patients have been divided into those with lower exposure and higher exposure, respectively. Results demonstrate that efficacy of KL1333 is driven by patients with high exposure (all having Total KL1333 AUC(0-tau) levels above 4500 h*ng/mL or C(min) above 100 ng/ml at day 10. Those with less effect had exposure levels below 3000 h*ng/mL or C(min) below 65 ng/ml at day 10. High exposure KL1333 group NeuroQol fatigue change from baseline was statistically significant better than placebo (Kruskal-Wallis test)

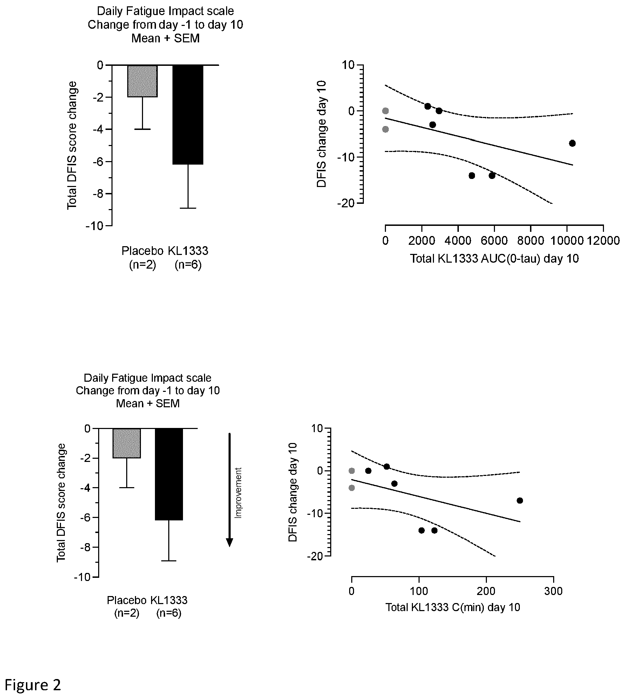

Figure 2 show data results from Daily Fatigue Impact Scale. Left graph demonstrate mean changes in KL1333 and placebo, with greater improvement (reduction of fatigue impacts on daily activities) in KL1333 group than placebo-treated. Right graph show correlation between effect sizes (change from baseline to day 10) and exposure levels, Total KL1333 AUC(0-tau) at day 10 (h*ng/mL).

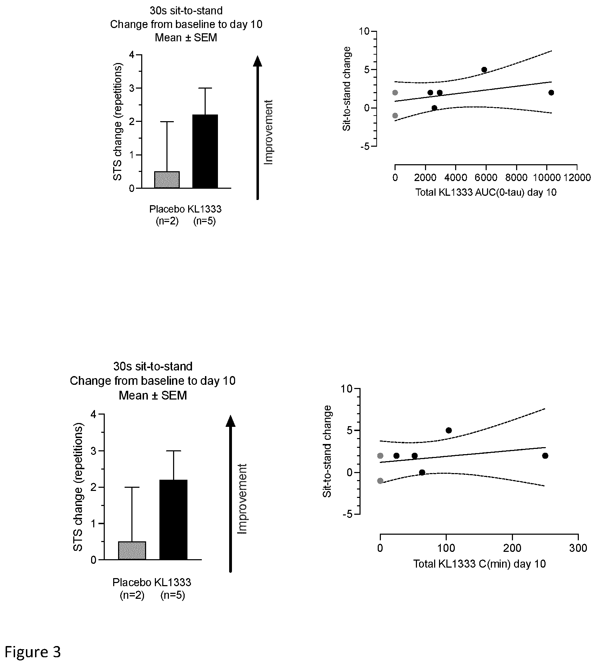

Figure 3 show data results from the 30s Sit-to-stand test, a functional test of high relevance to primary mitochondrial disease patients. Left graph demonstrate mean changes in KL1333 and placebo, with greater improvement (Increased number of sit to stand repetitions) in KL1333 group than placebo-treated. Right graph show correlation between effect sizes (change from baseline to day 10) and exposure levels, Total KL1333 AUC(0-tau) at day 10 (h*ng/mL).

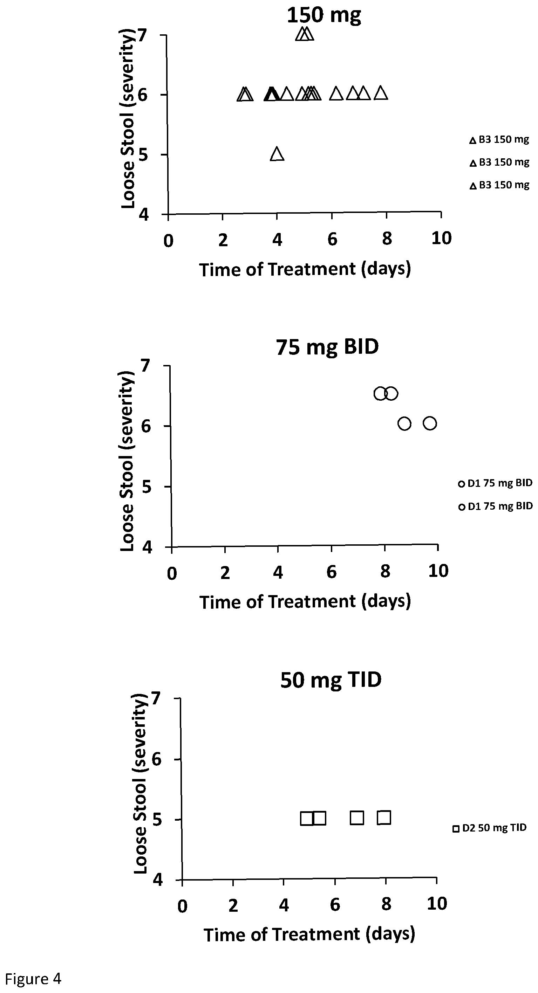

Figure 4 shows plotted loose stools episodes over time in the three cohorts. Severity of loose stool according to Bristol Stool chart is indicated on the y-axis, where grade 7 is most severe grade. The data demonstrate that both the frequency and severity of loose stool episodes are improved by dividing the daily dose into BID and TID dosing.

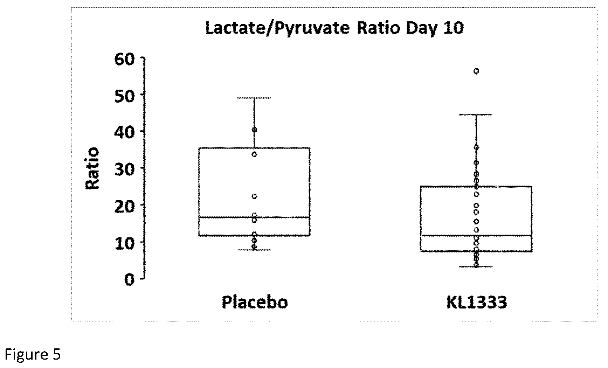

Figure 5. Healthy volunteers were treated with KL1333 (n=36) or placebo (n=14) once daily for 9 days prior to blood sampling for measurement of lactate and pyruvate concentrations and calculation of a lactate pyruvate ratio. Data for different doses of KL1333 were combined, including 50 mg QD, 50 mg TID, 75 mg QD, 75 mg BID, 150 mg QD and 250 mg QD.

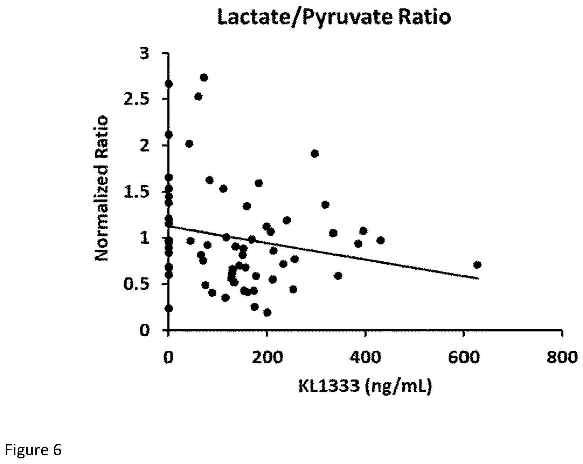

Figure 6. The blood lactate/pyruvate concentration ratio decreased with increasing concentration of KL1333 in plasma Day 10 after treatment with KL1333. Healthy volunteers were treated with KL1333 (n=36) or placebo (n=14) once daily for 9 days prior to blood sampling for measurement of lactate and pyruvate concentrations and calculation of a lactate pyruvate ratio. Data for different doses of KL1333 were combined from 7 cohorts consisting of two placebo and 6 KL1333 subjects, treated with 25 mg QD, 50 mg QD, 50 mg TID, 75 mg QD, 75 mg BID, 150 mg QD and 250 mg QD. Data were normalized against the cohort mean value for each cohort.

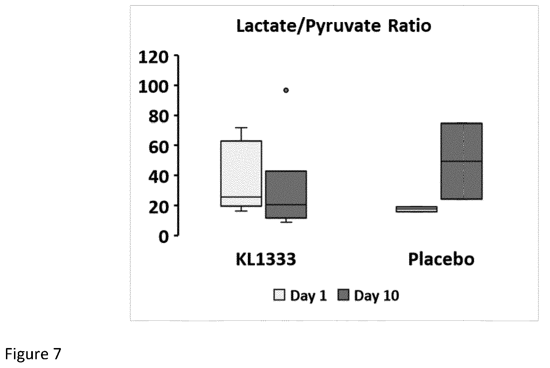

Figure 7. Lactate/Pyruvate ratio in PMD patients treated daily with 50 mg KL1333 (n=6) or placebo (n=2) for 10 days.

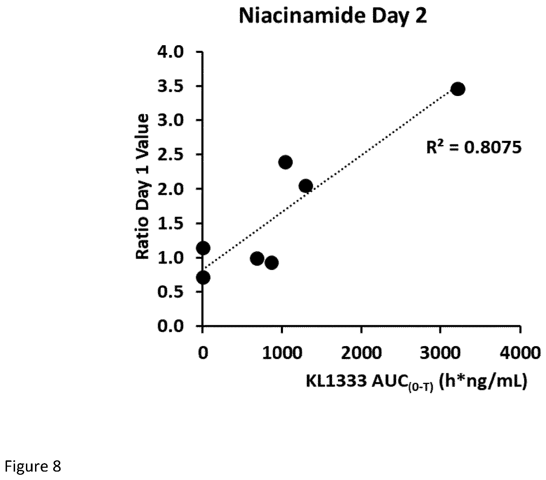

Figure 8. Serum niacinamide is an early biomarker of response to KL1333 treatment. PMD patients were treated with 50 mg KL1333 QD (n=6) or placebo (n=2). The data are expressed as the ratio of the niacinamide concentrations in Day 2 and Day 1 serum samples.

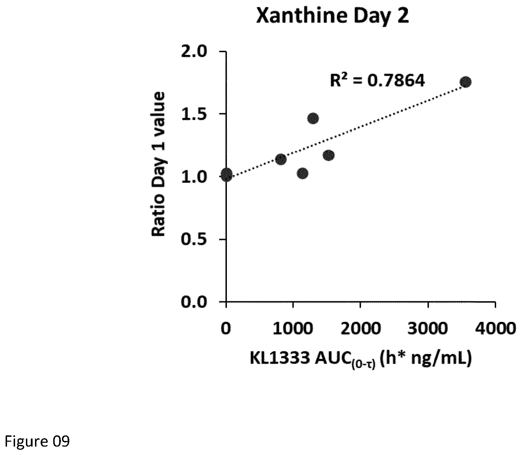

Figure 9. Serum xanthinse is an early biomarker of response to KL1333 treatment. PMD patients were treated with 50 mg KL1333 QD (n=6) or placebo (n=2). The data are expressed as the ratio of the xanthine concentrations in Day 2 and Day 1 serum samples.
Figure 10. DFIS fatigue score correlated with a low blood lactate/pyruvate ratio score after 10 days of treatment with 50 mg KL1333 (filled circles) or placebo (open circles).

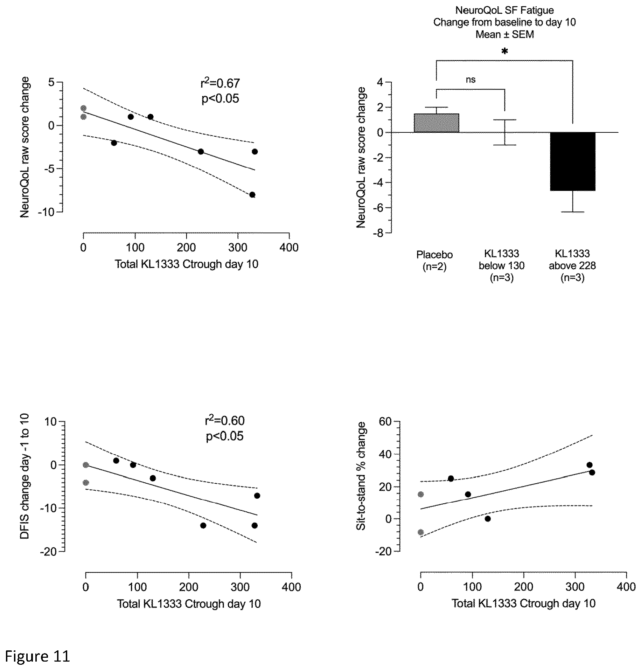

Figure 11 shows correlations between clinical outcomes and Total KL1333 Ctrough values. Upper left graph shows correlation between effect sizes in NeuroQol SF Fatigue raw score (change from baseline to day 10) and exposure levels, Total KL1333 Ctrough at day 10 (ng/mL) with a statistically significant correlation.

The upper right graph is mean data, where actively treated patients have been divided into those with lower exposure and higher exposure, respectively. Results demonstrate that efficacy of KL1333 is driven by patients with high exposure (all having Total KL1333 AUCtrough levels above 228 ng/ml at day 10. Those with less effect had exposure levels below 130 ng/ml at day 10. High exposure KL1333 group NeuroQol fatigue change from baseline was statistically significant better than placebo (Kruskal-Wallis test). Lower left graph show correlation between effect sizes in DFIS (change from day-1 to day 10) and exposure levels, Total KL1333 Ctrough at day 10 (ng/mL) with a statistically significant correlation. Lower right graph show correlation between effect sizes in 30s Sit-to-Stand (change from day-1 to day 10 in %) and exposure levels, Total KL1333 Ctrough at day 10 (ng/mL).

### EXAMPLES

Example 1 - A Randomised, Double-blind, Parallel-group, Placebo-controlled, Phase Ia/Ib, Multiple-site Study to Assess the Safety, Tolerability, Pharmacokinetics, and Pharmacodynamics of KL1333 after a Single Oral Dose and Multiple Ascending Oral Doses in Healthy Subjects and Patients with Primary Mitochondrial Disease

The primary objectives of the study are:
- To evaluate the safety and tolerability of a single oral dose, with and without food, and multiple ascending oral doses of KL1333 in healthy subjects
- To evaluate the safety and tolerability of multiple oral doses of KL1333 in patients with mitochondrial disease.

The secondary objectives of the study are:
- To determine the single oral dose plasma pharmacokinetics (PK) of KL1333 in healthy subjects, including the effect of food intake
- To determine the multiple oral dose plasma PK of KL1333 in healthy subjects and patients with mitochondrial disease.

The exploratory objectives of the study are:
- To explore the multiple-dose pharmacodynamics (PD) of KL1333 in healthy subjects and patients with mitochondrial disease using blood biomarkers
- To explore clinician- and patient-rated outcome assessments following multiple oral doses of KL1333 in patients with mitochondrial disease
- To collect blood samples for analysis of metabolomics following multiple oral doses of KL1333 in healthy subjects and patients with mitochondrial disease
- To assess the effect of KL1333 on electrocardiogram (ECG) parameters, including concentration-QT interval corrected for heart rate (QTc) analysis, in healthy subjects
- To collect blood samples for NAD(P)H:dehydrogenase [quinone]1 genotyping from healthy subjects who receive single or multiple oral doses of KL1333 and patients with mitochondrial disease who receive multiple oral doses of KL1333.

### Study design:

This will be a double-blind, randomised, placebo-controlled, single and multiple oral dose study conducted in 4 parts.
Part A: Single Ascending Dose (SAD) with food effect in healthy volunteers
Part B: Multiple Ascending Dose (MAD) in healthy volunteers
Part C: MAD in patients with primary mitochondrial disease (PMD)
Part D: Split dosing (dosing twice or three times daily) in healthy volunteer.

The four parts are described in details below.

### Part A:

Part A will comprise a randomised, single-dose, single-sequence, placebo-controlled study. Eight healthy subjects will be studied in a single cohort (Group A1).

Potential subjects will be screened to assess their eligibility to enter the study within 28 days prior to the first dose administration. Subjects will participate in 2 treatment periods. For each treatment period, subjects will reside at the Phase I clinical site from Days -1 to 3 (48 hours postdose). Subjects will return to the clinical site for outpatient visits on Days 4 and 5. There will be at least a 10-day washout between doses (from Period 1, Day 1 to Period 2, Day 1).

Six subjects will be randomised to receive 25 mg KL1333 and 2 subjects will be randomised to receive placebo, and subjects will receive the same treatment in both treatment periods. On Treatment Period 1, Day 1, subjects will receive a single oral dose of study drug following an overnight fast of at least 8 hours. On Treatment Period 2, Day 1, subjects will receive a single oral dose of study drug after consuming a standard high-fat breakfast. Following review of safety, tolerability, and PK data, up to 2 additional dose cohorts of healthy subjects may be added if needed to determine the study treatment for Part B. Additional single-dose cohorts may be enrolled based on data obtained from either Parts A or B. If additional cohorts are required, each cohort will consist of 8 subjects, with 6 subjects receiving KL1333 and 2 subjects receiving placebo, and will undergo a single treatment period. The dose level and dietary state for administration of KL1333 in these potential additional cohorts will be decided following review of data in Part A and any available data from Part B, and the dose level could be either less than or greater than 25 mg. The dose level will not exceed 600 mg, and the predicted exposure following a single dose in any subject in Part A will not exceed an area under the plasma concentration-time curve [AUC] from time zero to 24 hours postdose [AUC₀₋₂₄] of 51,800 ng.h/mL for derived total KL1333.

Subjects will return for a Follow-up visit on Day 6, 5 days after their final dose.

### Part B:

Part B will comprise a randomised, multiple-dose, sequential-group, placebo-controlled study. Sixteen healthy subjects will be studied in 2 cohorts (Groups B1 and B2), with each cohort consisting of 8 subjects. Part B may start after completion of Group A1, at a dose equal to or less than given in Part A.

Potential subjects will be screened to assess their eligibility to enter the study within 28 days prior to the first dose administration. All subjects will participate in 1 treatment period and will reside at the Phase I clinical site from Days -1 to 12 (48 hours post final dose). Subjects will return to the clinical site for outpatient visits on Days 13 and 14.

On Day 1, 6 subjects will be randomised to receive KL1333 and 2 subjects will be randomised to receive placebo. The preliminary planned doses of KL1333 for Groups B1 and B2 are 25 and 50 mg, respectively, once daily (QD) on Days 1 to 10. Dose levels, dose frequency, and dietary state will be confirmed following review of safety, tolerability, and PK data from Part A and ongoing data from Part B. Additionally, a dose selection conference meeting will be held before each cohort in Part B where blinded data from the previous cohort will be reviewed before a decision is made about proceeding to the next cohort. Following review of safety, tolerability, and PK data, up to 3 additional dose cohorts of healthy subjects may be added to further explore the PK, safety, and tolerability of KL1333. If additional cohorts are required, each cohort will consist of 8 subjects, with 6 subjects receiving KL1333 and 2 subjects receiving placebo. The dose level will not exceed 600 mg, and the predicted exposure following multiple daily dose administration in any subject in Part B will not exceed an AUC₀₋₂₄ of 51,800 ng.h/mL for derived total KL1333. There will be a minimum of 6 days between dose escalations for each cohort (between the last dose of one cohort and the first dose of the next cohort).

Subjects will return for a Follow-up visit on Day 15, 5 days after their final dose.

### Part C:

Part C will comprise a randomised, multiple-dose, single-group, placebo-controlled study. A total of 8 patients diagnosed with any mitochondrial disease will be enrolled in this part of the study. Part C may start after the dose selection conference has been completed for the final cohort of Part B, at a daily dose no higher than the highest well-tolerated dose in Part B.

Potential study patients will be screened to assess their eligibility to enter the study within 75 days prior to the first dose administration. Patients will reside at the clinical site, or nearby the clinical site at a hotel recommended by the clinical site, from Days -1 to 2 and Days 10 to 11. Patients will return to the clinical site for outpatient visits on Days 4 and 8. Patients will be randomised on Day 1.

Two patients will be initially dosed, with 1 patient receiving KL1333 and 1 patient receiving placebo. If there are no safety or tolerability concerns in these patients following the Day 4 visit, the remaining 6 patients, with 5 patients receiving KL1333 and 1 patient receiving placebo, will be enrolled on a rolling basis. In the event there are safety concerns following completion of the 2 sentinel patients without meeting stopping criteria, the Sponsor may add intermediate cohorts if the safety evaluation in intermediate cohorts will be needed. The dosing schedule of intermediate cohorts will be the same as that of the planned cohort. The schedule of safety assessments of intermediate cohorts will be the same as that of the planned cohort as a general rule. Whether or not to add safety assessments in the intermediate cohorts will be determined by the Sponsor. It is planned for patients to receive study drug QD on Days 1 to 10. Dose levels, dose frequency, and dietary state will be confirmed following review of safety, tolerability, and PK data from Part B, and unless deemed very unfavourable for the conduct of the study, the patients will not be required to be fasting prior to dosing. Study drug will be administered by clinical site staff when the patients are resident at the clinical site or return for outpatient visits. On all other days, patients will record drug administration and any concomitant medications in a diary that will be provided to each patient. Diaries will be reviewed and checked for compliance during the outpatient visits and as part of the check-in procedures on Day 10. Clinical symptoms that occur while patients are not resident at the site will be collected by the site using standard adverse event (AE) reporting procedures.

Patients will return for a Follow-up visit on Day 15, 5 days after their final dose.

### Part D:

Part D will comprise a randomised, multiple-dose, placebo-controlled study. Sixteen healthy subjects will be studied in 2 cohorts (Groups D1 and D2), with each cohort consisting of 8 subjects. Part D will start after completion of Part B, and the Part D groups may be run in parallel.

Potential subjects will be screened to assess their eligibility to enter the study within 35 days prior to the first dose administration. All subjects will participate in 1 treatment period and will reside at the Phase I clinical site from Days -1 to 12 (48 hours post final dose). Subjects will return to the clinical site for outpatient visits on Days 13 and 14.

On Day 1, 6 subjects will be randomised to receive KL1333 and 2 subjects will be randomised to receive placebo. The doses of KL1333 for Groups D1 and D2 are 75 mg twice daily (BID) and 50 mg 3 times daily (TID), respectively, on Days 1 to 10 with a single dose administration on Day 10.

Subjects will return for a Follow-up visit on Day 15, 5 days after their final dose.

### Investigational products, dose, and mode of administration:

Test products: 25 and 100 mg KL1333 encapsulated tablets and matching encapsulated placebo tablets. Placebo tablets are identical to test products regarding appearance, shape and weight.

KL1333 Drug Product is an immediate release tablet intended for oral administration.

Proposed dose level for Part A: 25 mg KL1333 or placebo administered once in the fasted state and once in the fed state.

Proposed dose levels for Part B: 25 and 50 mg KL1333 or placebo QD for 10 days. The dose level, dosing frequency, and dietary state for Part B will be decided, in consultation with the Sponsor, on the basis of data from Part A of the study and emerging interim data from Part B.

Patients in Part C will be administered KL1333 or placebo QD for 10 days. The dose level, dosing frequency, and dietary state for Part C will be decided, in consultation with the Sponsor, on the basis of data from Part B of the study.

Dose levels for Part D: 75 mg BID and 50 mg TID KL1333 or placebo for 10 days with a single dose administration on Day 10. The first dose on Days 1 and 7 and the dose on Day 10 will be administered in the fasted state. All other doses can be administered without regard to food.

The dose level will not exceed 600 mg, and the predicted exposure in any subject in any cohort in this study will not exceed an AUC₀₋₂₄ of 51,800 ng.h/mL for derived total KL1333 using the revised bioanalytical method that measures KL1333 as the sum of parent KL1333, de-conjugated glucuronidated KL1333 metabolites, and sulphated KL1333 metabolites.

### Endpoints:

### Pharmacokinetics:

Blood samples for the analysis of plasma concentrations of KL1333 will be collected, and PK parameters will be derived by noncompartmental analysis.

For Part A, the PK parameters will include:
- AUC from time zero to infinity (AUC_{0-∞})
- AUC₀₋₂₄
- AUC from time zero to the time of the last quantifiable concentration (AUC₀₋ₜₗₐₛₜ)
- Cₘₐₓ
- time of the Cₘₐₓ (Tₘₐₓ)
- apparent plasma terminal elimination half-life (t_{1/2})
- mean residence time (MRT)
- apparent total plasma clearance (CL/F)
- apparent volume of distribution during the terminal phase (V_{z}/F).

For Parts B through D, the PK parameters will include:
- AUC_{0-∞} (Day 1 only)
- AUC over a dosing interval (AUC_{0-τ}; Days 1 and 10)
- temporal change parameter (TCP; AUC_{0-τ}/AUC_{0-∞})
- Cₘₐₓ
- minimum observed plasma concentration (Cₘᵢₙ)
- Tₘₐₓ
- t_{1/2}
- MRT on Days 1 and 10
- CL/F on Days 1 and 10
- V_{z}/F on Days 1 and 10
- observed accumulation ratio based on AUC_{0-τ} (RA_{AUC})
- observed accumulation ratio based on Cₘₐₓ (RA_{Cmax})
- peak-to-trough ratio (PTR).

Other PK parameters will be calculated if appropriate.

### Pharmacodynamics:

For Parts B through D, blood biomarker assessments will include:
- nicotinamide adenine dinucleotide (oxidized form; NAD⁺)/nicotinamide adenine dinucleotide (reduced form; NADH) concentrations and ratio
- fibroblast growth factor-21 (FGF21)
- growth/differentiation factor-15 (GDF15)
- lactate/pyruvate concentrations and ratio.

For Part C, blood biomarkers assessments will also include:
- glucose
- glycated albumin/albumin concentrations and ratio.

For Part C, clinician- and patient-rated assessments will include:
- Newcastle Mitochondrial Disease Adult Scale
- Clinician Global Impression
- Patient Global Impression-Improvement
- Daily Fatigue Impact Severity
- Quality of Life in Neurological Disorders Fatigue Short Form
- 30 Second Sit-to-Stand Test

### Results - Efficacy in patients with primary mitochondrial disease

Patient-rated outcome assessments following multiple oral doses of KL1333 in patients with mitochondrial disease.

6 patients with genetically confirmed primary mitochondrial disease were given active treatment with 50 mg KL1333 once daily for 10 days. 2 patients were given placebo.

Results from three main clinical outcome assessments in primary mitochondrial disease patients are displayed in Figure 1. Data show mean changes from baseline to last day of dosing (day 10)
- Quality of Life in Neurological Disorders Fatigue Short Form
- Daily Fatigue Impact Scale (D-FIS)
- 30 Second Sit-to-Stand Test (test for muscle strength)

The lower graphs are same data, but actively treated patients have been divided into those with lower exposure and higher exposure, respectively. Results demonstrate that efficacy of KL1333 is driven by patients with high exposure (all having Total KL1333 AUC(0-tau) levels above 4500 h*ng/mL, C(min) above 100 ng/mL, and/or Ctrough above 228 ng/mL at day 10). Those with less effect had exposure levels below 3000 h*ng/mL, C(min) below 65 ng/mL, and/or Ctrough below 130 ng/mL at day 10.

The results are given in Figures 1-3 and show that patients given KL1333 showed
i) a marked improvement in fatigue,
ii) a marked improvement in muscle strength
iii) strong response in effect compared with placebo
and all effects were obtained with only 10 days of dosing and the dose was 50 mg once daily.

Figure 1 show data results from NeuroQoL Short Form Fatigue. Debilitating fatigue is considered the most important symptom to improve from mitochondrial disease patients.

Left graph demonstrate mean changes in KL1333 and placebo, with improvement in KL1333 group, but not placebo-treated. Middle graph show correlation between effect sizes (change from baseline to day 10) and exposure levels, Total KL1333 AUC(0-tau) or C(min) at day 10 (h*ng/mL). The right graph is the same data, but actively treated patients have been divided into those with lower exposure and higher exposure, respectively. Results demonstrate that efficacy of KL1333 is driven by patients with high exposure (all having Total KL1333 AUC(0-tau) levels above 4500 h*ng/mL at day 10. Or C(min) above 100 ng/ml. Those with less effect had exposure levels below 3000 h*ng/mL at day 10. Or C(min) below 65 ng/ml High exposure KL1333 group NeuroQol fatigue change from baseline was statistically significant better than placebo (Kruskal-Wallis test).

Figure 2 show data results from Daily Fatigue Impact Scale, and Figure 4 show data results from the 30s Sit-to-stand test, a functional test of high relevance to primary mitochondrial disease patients

### Tolerability improvement

The safety and tolerability of KL1333 in healthy volunteers was explored at multiple ascending doses in part B and D of the study. Part D specifically explored if tolerability was improved by dividing the daily dose into two and three administrations respectively. Cohorts B3, D1 and D2 all recieved total daily doses of 150 mg for 10 days. Cohort B3 received 150 mg once daily (QD), cohort D1 received 75 mg twice daily (BID), and cohort D2 50 mg three times daily (TID). In Figure 4 are plotted loose stools episodes over time in the three cohorts. Severity of loose stool according to Bristol Stool chart is indicated on the y-axis, where grade 7 is most severe grade. The data demonstrate thate bothe the frequency and severity of loose stool episodes are improved by dividing the dose into BID and TID dosing.

### Exposure

Table below show exposure levels of Total KL1333 at day 10 in healthy volunteers in part D. All subject received steady-state concentrations exceeding 3900 h*ng/mL at day 10, and none were below 3000 h*ng/mL at day 10.

| |
|---|
| Population: Pharmacokinetic Matrix: Plasma; Analyte: Total KL1333; Profile Day: 10 |

| Parameter | Statistic | 75 mg KL1333 BID (N = 6) | 50 mg KL1333 TID (N = 6) |
|---|---|---|---|
| | | | |
| AUC₀₋₂₄ (h*ng/mL) | Geometric Mean | 5130 | 6580 |
| | Min | 4430 | 3920 |
| | Max | 5530 | 9890 |

All of the cohorts confirmed the safe profile of KL1333 with no serious adverse events or safety signals seen in the study. KL1333 was generally well tolerated across patients and healthy volunteers with the main dose-limiting tolerability of gastrointestinal side effects, an effect that was improved by dividing the daily dose. The pharmacokinetics profile was similar in healthy volunteers and patients

### Example 2 - Biomarkers (not part of the invention)

KL1333 showed a trend of decreasing blood lactate/pyruvate ratio after 10 days of treatment of healthy volunteers with KL1333 (Figure 5).

The blood lactate pyruvate ratio was lowest in individuals with a plasma concentration of KL1333 exceeding 100 ng/mL prior to taking blood samples for analysis (Figure 6).

In a study of patients with PMD treated daily with KL1333 for 10 days, the lactate/pyruvate ratio had decreased on Day 10 compared to Day 1 in the KL1333 treated subjects but not in placebo treated subjects (Figure 7).

Niacinamide, a metabolite in the pathway of nicotinamide dinucleotide synthesis increased after one day of treatment of PMD patients with KL1333 (Figure 8). The increase was strongest in the patients with the highest exposure to KL1333.

Xanthine, a metabolite in the pathway of purine metabolism resulting from oxidation of hypoxanthine with NAD⁺ as cofactor, increased after one day of treatment of PMD patients with KL1333 (Figure 9). The increase was strongest in the patients with the highest exposure to KL1333.

In conclusion, blood lactate/pyruvate ratio, niacinamide and/or xanthine may be used as biomarkers of KL1333 treatment effect in PMD patients. To that end, it was found that patients with the strongest decrease in the DFIS fatigue score Day 10 of KL1333 treatment also a low lactate/pyruvate ratio in serum (Figure 10).

## Claims

1. KL1333 for use in the treatment of fatigue, wherein said fatigue is a manifestation associated with a mitochondrial disease.

2. KL1333 for use according to claim 1, wherein fatigue is physical fatigue, mental fatigue, neurological fatigue or chronic fatigue.

3. KL1333 for use in the treatment of muscle weakness, wherein said muscle weakness is a manifestation associated with a mitochondrial disease.

4. KL1333 for use according to any one of claims 1 to 3, wherein the mitochondrial disease is selected from the following:
• Alpers Disease (Progressive Infantile Poliodystrophy)
• Amyotrophic lateral sclerosis (ALS)
• Autism
• Barth syndrome (Lethal Infantile Cardiomyopathy)
• Beta-oxidation Defects
• Bioenergetic metabolism deficency
• Carnitine-Acyl-Carnitine Deficiency
• Carnitine Deficiency
• Creatine Deficiency Syndromes (Cerebral Creatine Deficiency Syndromes (CCDS) includes: Guanidinoaceteate Methyltransferase Deficiency (GAMT Deficiency), L- Arginine:Glycine Am id i notransferase Deficiency (AGAT Deficiency), and SLC6A8-Related Creatine Transporter Deficiency (SLC6A8 Deficiency).
• Co-Enzyme Q10 Deficiency
• Complex I Deficiency (NADH dehydrogenase (NADH-CoQ reductase deficiency)
• Complex II Deficiency (Succinate dehydrogenase deficiency)
• Complex III Deficiency (Ubiquinone-cytochrome c oxidoreductase deficiency)
• Complex IV Deficiency/COX Deficiency (Cytochrome c oxidase deficiency is caused by a defect in Complex IV of the respiratory chain)
• Complex V Deficiency (ATP synthase deficiency)
• COX Deficiency
• CPEO (Chronic Progressive External Ophthalmoplegia Syndrome)
• CPT I Deficiency
• CPT II Deficiency
• Friedreich's ataxia (FRDA or FA)
• Glutaric Aciduria Type II
• KSS (Kearns-Sayre Syndrome)
• Lactic Acidosis
• LCAD (Long-Chain Acyl-CoA Dehydrogenase Deficiency)
• LCHAD
• Leigh Disease or Syndrome (Subacute Necrotizing Encephalomyelopathy)
• LHON (Leber's hereditary optic neuropathy)
• Luft Disease
• MCAD (Medium-Chain Acyl-CoA Dehydrogenase Deficiency)
• MELAS (Mitochondrial Encephalomyopathy Lactic Acidosis and Strokelike Episodes)
• MERRF (Myoclonic Epilepsy and Ragged-Red Fiber Disease)
• MIDD (Maternally inherited Diabetes and Deafness)
• MIRAS (Mitochondrial Recessive Ataxia Syndrome)
• Mitochondrial Cytopathy
• Mitochondrial DNA Depletion
• Mitochondrial Encephalopathy includes: Encephalomyopathy, Encephalomyelopathy
• Mitochondrial Myopathy
• MNGIE (Myoneurogastointestinai Disorder and Encephalopathy)
• NARP (Neuropathy, Ataxia, and Retinitis Pigmentosa)
• Neurodegenerative disorders associated with Parkinson's, Alzheimer's or Huntington's disease
• Pearson Syndrome
• Pyruvate Carboxylase Deficiency
• Pyruvate Dehydrogenase Deficiency
• POLG Mutations • Respiratory Chain Deficiencies
• SCAD (Short-Chain Acyl-CoA Dehydrogenase Deficiency)
• SCHAD ( Short Chain L-3-Hydroxyacyl-CoA Dehydrogenase (SCHAD) Deficiency, also referred to as 3-Hydroxy Acyl CoA Dehydrogenase Deficiency HADH
• VLCAD (Very Long-Chain Acyl-CoA Dehydrogenase Deficiency)
• Diabetes
• Acute starvation
• Endotoxemia
• Sepsis
• Systemic inflammation response syndrome (SIRS)
• Multiple organ failure.

5. KL1333 for use according to any one of claims 1 to 4, wherein the mitochondrial disease is caused by a Complex I defect.

6. KL1333 for use according to any one of the preceding claims, wherein a dose of KL1333 is administered once, twice or three times daily.

7. KL1333 for use according to any one of the preceding claims, wherein the treatment is continued for at least 2 days such as at least 5 days, at least 10 days, at least 4 weeks or at least 2 months.

8. KL1333 for use in a dosage regime for the treatment of one or more of fatigue, or muscle weakness,
wherein said fatigue or muscle weakness is a manifestation associated with a mitochondrial disease the dosage regime comprising:
i) administering to a subject suffering from fatigue, muscle weakness or a mitochondrial disease in an amount from 25 mg to 100 mg KL1333 daily for a time period of from 2 to 10 days to obtain steady state concentrations of KL1333 in the blood,
ii) measuring the blood concentration of KL1333 expressed as AUC (area under the curve) or Cmin and if AUC is below 3,000 h*ng/mL or Cmin is 65 ng/mL or less,
iii) adjusting the daily dose to obtain steady state concentrations of KL1333 in the blood corresponding to an AUC (area under the curve) of at least 3,000 h*ng/mL or Cmin of at least 65 ng/ml, or such that AUC is at least 3,500 h*ng/mL or Cmin is at least 77 ng/mL, or such that AUC is at least 4,000 h*ng/mL or Cmin is at least 88 ng/mL, or such that AUC is at least 4,500 h*ng/mL or Cmin is at least 100 ng/mL or such that AUC is in a range of from 4,000 to 12,000 h*ng/mL or Cmin is in a range of from 88 ng/mL to 275 ng/mL, at day 10 after adjustment of the dose.

9. KL1333 for use according to claim 8, wherein the dosage regime is administered orally.

10. KL 1333 for use according to any of claims 1 to 9, wherein a ratio between the blood concentration of lactate (mM) and the blood concentration of pyruvate (mM) (lactate/pyruvate ratio) is used as biomarker of KL1333 treatment effect, wherein a decrease in ratio after start of the treatment indicates that the treatment is effective.

## Patentansprüche

1. KL1333 zur Verwendung bei der Behandlung von Ermüdung, wobei die Ermüdung eine mit einer mitochondrialen Erkrankung assoziierte Manifestation ist.

2. KL1333 zur Verwendung nach Anspruch 1, wobei es sich bei der Ermüdung um körperliche Ermüdung, geistige Ermüdung, neurologische Ermüdung oder chronische Ermüdung handelt.

3. KL1333 zur Verwendung bei der Behandlung von Muskelschwäche, wobei die Muskelschwäche eine mit einer mitochondrialen Erkrankung assoziierte Manifestation ist.

4. KL1333 zur Verwendung nach einem der Ansprüche 1 bis 3, wobei die mitochondriale Erkrankung aus Folgendem ausgewählt ist:
• Alpers-Krankheit (progressive infantile Poliodystrophie)
• Amyotrophe Lateralsklerose (ALS)
• Autismus
• Barth-Syndrom (letale infantile Kardiomyopathie)
• Beta-Oxidation-Defekte
• Bioenergetischer Stoffwechselmangel
• Carnitin-Acyl-Carnitin-Mangel
• Carnitin- und Kreatinmangelsyndrome (Cerebral Creatine Deficiency Syndromes, CCDS), einschließlich:
Guanidinoacetat-Methyltransferase-Mangel (GAMT-Mangel), L-Arginin:Glycin-Amidinotransferase-Mangel (AGAT-Mangel) und SLC6A8-bedingter Kreatin-Transporter-Mangel (SLC6A8-Mangel).
• Coenzym-Q10-Mangel
• Komplex-I-Mangel (NADH-Dehydrogenase (NADH-CoQ-Reduktase-Mangel) Komplex-II-Mangel (Succinat-Dehydrogenase-Mangel)
• Komplex-III-Mangel (Ubiquinon-Cytochrom-C-Oxidoreduktase-Mangel)
• Komplex-IV-Mangel/COX-Mangel (Cytochrom-C-Oxidase-Mangel wird durch einen Defekt im Komplex-IV der Atmungskette verursacht)
• Komplex-V-Mangel (ATP-Synthase-Mangel)
• COX-Mangel
• CPEO (chronisches progressives externes Ophthalmoplegiesyndrom)
• CPT-1-Mangel
• CPT-2-Mangel
• Friedreich-Ataxie (FRDA oder FA)
• Glutarazidurie Typ II
• KSS (Kearns-Sayre-Syndrom)
• Laktatazidose
• LCAD (Langkettige-Acyl-CoA-Dehydrogenase-Mangel)
• LCHAD
• Leigh-Krankheit oder -Syndrom (subakute nekrotisierende Enzephalomyelopathie)
• LHON (Lebersche hereditäre Optikusneuropathie)
• Luftkrankheit
• MCAD (Mittelkettige-Acyl-CoA-Dehydrogenase-Mangel)
• MELAS (mitochondriale Enzephalomyopathie, Laktatazidose und schlaganfallähnliche Episoden)
• MERRF (myoklonische Epilepsie und Ragged-Red-Fiber-Krankheit)
• MIDD (mütterlich vererbter Diabetes und Taubheit)
• MIRAS (mitochondriales rezessives Ataxiesyndrom)
• Mitochondriale Zytopathie
• Mitochondriale DNA-Depletion
• Mitochondriale Enzephalopathie einschließlich: Enzephalomyopathie, Enzephalomyelopathie, mitochondriale Myopathie
• MNGIE (myoneurogastrointestinale Erkrankung und Enzephalopathie)
• NARP (Neuropathie, Ataxie und Retinitis pigmentosa)
• Neurodegenerative Erkrankungen im Zusammenhang mit Parkinson, Alzheimer oder Morbus Huntington
• Pearson-Syndrom
• Pyruvat-Carboxylase-Mangel
• Pyruvat-Dehydrogenase-Mangel
• POLG-Mutationen und Atmungskettendefekte
• SCAD (Kurzkettige-Acyl-CoA-Dehydrogenase-Mangel)
• SCHAD (Kurzkettige-L-3-Hydroxyacyl-CoA-Dehydrogenase-Mangel (SCHAD), auch als 3-Hydroxy-Acyl-CoA-Dehydrogenase-Mangel (HADH) bezeichnet)
• VLCAD (Sehr langkettige-Acyl-CoA-Dehydrogenase-Mangel)
• Diabetes
• Akutes Hungern
• Endotoxämie
• Sepsis
• Systemisches Entzündungsreaktionssyndrom (SIRS)
• Multiorganversagen.

5. KL1333 zur Verwendung nach einem der Ansprüche 1 bis 4, wobei die mitochondriale Erkrankung durch einen Komplex-I-Defekt verursacht wird.

6. KL1333 zur Verwendung nach einem der vorstehenden Ansprüche, wobei eine Dosis von KL1333 einmal, zweimal oder dreimal täglich verabreicht wird.

7. KL1333 zur Verwendung nach einem der vorstehenden Ansprüche, wobei die Behandlung für mindestens 2 Tage, wie zum Beispiel mindestens 5 Tage, mindestens 10 Tage, mindestens 4 Wochen oder mindestens 2 Monate, fortgesetzt wird.

8. KL1333 zur Verwendung in einem Dosierungsschema zur Behandlung von Ermüdung und/oder Muskelschwäche,
wobei die Ermüdung oder Muskelschwäche eine mit einer mitochondrialen Erkrankung assoziierte Manifestation ist, wobei das Dosierungsschema umfasst:
i) Verabreichen von KL1333 in einer Menge von 25 mg bis 100 mg täglich über einen Zeitraum von 2 bis 10 Tagen an einen Probanden, der an Ermüdung, Muskelschwäche oder einer mitochondrialen Erkrankung leidet, um Steady-State-Konzentrationen von KL1333 im Blut zu erreichen,
ii) Messen der Blutkonzentration von KL1333, ausgedrückt als AUC (Fläche unter der Kurve) oder Cmin, und wenn AUC unter 3.000 h*ng/ml liegt oder Cmin 65 ng/ml oder weniger beträgt,
iii) Anpassen der Tagesdosis, um Steady-State-Konzentrationen von KL1333 im Blut zu erhalten, die einer AUC (Fläche unter der Kurve) von mindestens 3.000 h*ng/ml oder Cmin von mindestens 65 ng/ml entsprechen, oder so, dass die AUC mindestens 3.500 h*ng/ml beträgt oder Cmin mindestens 77 ng/ml beträgt, oder so, dass die AUC mindestens 4.000 h*ng/ml beträgt oder Cmin mindestens 88 ng/ml beträgt, oder so, dass die AUC mindestens 4.500 h*ng/ml beträgt oder Cmin mindestens 100 ng/ml beträgt, oder so, dass die AUC in einem Bereich von 4.000 bis 12.000 h*ng/ml oder Cmin in einem Bereich von 88 ng/ml bis 275 ng/ml liegt, an Tag 10 nach der Dosisanpassung.

9. KL1333 zur Verwendung nach Anspruch 8, wobei das Dosierungsschema oral verabreicht wird.

10. KL1333 zur Verwendung nach einem der Ansprüche 1 bis 9, wobei ein Verhältnis zwischen der Blutkonzentration von Lactat (mM) und der Blutkonzentration von Pyruvat (mM) (Lactat/Pyruvat-Verhältnis) als Biomarker der Behandlungswirkung von KL1333 verwendet wird, wobei eine Abnahme des Verhältnisses nach Beginn der Behandlung darauf hindeutet, dass die Behandlung wirksam ist.

## Revendications

1. KL1333 pour une utilisation dans le traitement de la fatigue, dans lequel ladite fatigue est une manifestation associée à une maladie mitochondriale.

2. KL1333 pour une utilisation selon la revendication 1, dans lequel la fatigue est une fatigue physique, une fatigue mentale,
une fatigue neurologique ou une fatigue chronique.

3. KL1333 pour une utilisation dans le traitement de la faiblesse musculaire, dans lequel ladite faiblesse musculaire est une manifestation associée à une maladie mitochondriale.

4. KL1333 pour une utilisation selon l'une quelconque des revendications 1 à 3, dans lequel la maladie mitochondriale est sélectionnée parmi ce qui suit :
• la maladie d'Alpers (poliodystrophie infantile progressive)
• la sclérose latérale amyotrophique (SLA)
• l'autisme
• le syndrome de Barth (cardiomyopathie infantile létale)
• des déficits de la bêta-oxydation
• un déficit du métabolisme bioénergétique
• un déficit en carnitine-acylcarnitine
• les syndromes de déficit de carnitine et en créatine (syndromes cérébraux de déficit en créatine, CDDS) incluent :
• un déficit en guanidinoacétate méthyltransférase (déficit en GAMT), déficit en L-arginine:glycine amidinotransférase (déficit en AGAT) et déficit du transporteur de la créatine lié à SLC6A8 (déficit en SLC6A8)
• un déficit en coenzyme Q10
• un déficit du complexe I (déficit en NADH déshydrogénase (déficit en NADH-CoQ réductase)), un déficit du complexe II (déficit en succinate déshydrogénase)
• un déficit du complexe III (déficit en ubiquinone-cytochrome C oxyréductase), un déficit du complexe IV/déficit en COX (un déficit en cytochrome C oxydase est causé par une anomalie du complexe IV dans la chaîne respiratoire)
• un déficit du complexe V (déficit en ATP synthase)
• un déficit en COX
• un CPEO (syndrome d'ophtalmoplégie externe chronique progressive)
• un déficit en CPT I
• un déficit en CPT II
• une ataxie de Friedreich (FRDA ou FA)
• une acidurie glutarique de type Il
• un KSS (syndrome de Kearns-Sayre)
• une acidose lactique
• un LCAD (déficit en acyl-CoA déshydrogénase à longue chaîne)
• un LCHAD
• une maladie ou un syndrome de Leigh (encéphalomyélopathie nécrosante subaiguë)
• une NOHL (neuropathie optique héréditaire de Leber)
• une maladie de Luft
• un MCAD (déficit en acyl-CoA déshydrogénase à chaîne moyenne)
• une MELAS (encéphalomyopathie mitochondriale, acidose lactique et épisodes pseudo-accident vasculaire cérébral), une MERRF (épilepsie myoclonique et maladie des fibres rouges déchiquetées)
• un MIDD (diabète et surdité d'origine maternelle)
• un MIRAS (syndrome d'ataxie récessive mitochondriale)
• une cytopathie mitochondriale
• une déplétion de l'ADN mitochondrial
• une encéphalopathie mitochondriale inclut : une encéphalomyopathie, une encéphalomyélopathie, une myopathie mitochondriale
• une MNGIE (encéphalomyopathie mitochondriale neurogastrointestinale)
• une NARP (neuropathie, ataxie et rétinite pigmentaire)
• des troubles neurodégénératifs associés à la maladie de Parkinson, d'Alzheimer ou de Huntington
• le syndrome de Pearson
• un déficit en pyruvate carboxylase
• un déficit en pyruvate déshydrogénase
• des mutations de POLG, des déficits de la chaîne respiratoire
• un SCAD (déficit en acyl-CoA déshydrogénase à chaîne courte)
• un SCHAD (déficit en L-3-hydroxyacyl-CoA déshydrogénase à chaîne courte (SCHAD)), également appelé déficit en 3-hydroxyacyl-CoA déshydrogénase HADH
• un VLCAD (déficit en acyl-CoA déshydrogénase à très longue chaîne)
• un diabète
• une inanition aiguë
• une endotoxémie
• une sepsie
• un syndrome de réponse inflammatoire systémique (SRIS)
• un syndrome de défaillance multiviscérale.

5. KL1333 pour une utilisation selon l'une quelconque des revendications 1 à 4, dans lequel la maladie mitochondriale est causée par une anomalie du complexe I.

6. KL1333 pour une utilisation selon l'une quelconque des revendications précédentes, dans lequel une dose de KL1333 est administrée une fois, deux fois ou trois fois par jour.

7. KL1333 pour une utilisation selon l'une quelconque des revendications précédentes, dans lequel le traitement est poursuivi pendant au moins 2 jours tels qu'au moins 5 jours, au moins 10 jours, au moins 4 semaines ou au moins 2 mois.

8. KL1333 pour une utilisation dans un régime posologique pour le traitement d'une ou plusieurs parmi la fatigue ou la faiblesse musculaire,
dans lequel ladite fatigue ou faiblesse musculaire est une manifestation associée à une maladie mitochondriale, le régime posologique comprenant :
i) l'administration à un sujet souffrant de fatigue, de faiblesse musculaire ou d'une maladie mitochondriale en une quantité quotidienne de 25 mg à 100 mg de KL1333 pendant une période de 2 à 10 jours pour obtenir des concentrations à l'état d'équilibre de KL1333 dans le sang,
ii) la mesure de la concentration sanguine de KL1333 exprimée en ASC (aire sous la courbe) ou en Cmin et si l'ASC est inférieure à 3 000 h*ng/ml ou la Cmin est inférieure ou égale à 65 ng/ml,
iii) l'ajustement de la dose quotidienne pour obtenir des concentrations à l'état d'équilibre de KL1333 dans le sang correspondant à
une ASC (aire sous la courbe) d'au moins 3 000 h*ng/ml ou une Cmin d'au moins 65 ng/ml, ou telle que l'ASC est d'au moins 3 500 h*ng/ml ou que la Cmin est d'au moins 77 ng/ml, ou telle que
l'ASC est d'au moins 4 000 h*ng/ml ou la Cmin est d'au moins 88 ng/ml, ou telle que l'ASC est d'au moins 4 500 h*ng/ml ou la Cmin est d'au moins 100 ng/mL ou telle que l'ASC est dans une gamme de 4 000 à 12 000 h*ng/ml ou la Cmin est dans une plage de 88 ng/ml à 275 ng/ml, au jour 10 après l'ajustement de la dose.

9. KL1333 pour une utilisation selon la revendication 8, dans lequel le régime posologique est administré par voie orale.

10. KL1333 pour une utilisation selon l'une quelconque des revendications 1 à 9, dans lequel un rapport entre la concentration sanguine de lactate (mM) et la concentration sanguine de pyruvate (mM) (rapport lactate/pyruvate) est utilisé comme biomarqueur de l'effet de traitement du KL1333, dans lequel une diminution du rapport après le début du traitement indique que le traitement est efficace.
